# EUROPEAN PATENT SPECIFICATION

(11) **EP 1 890 735 B1**
(45) Date of publication and mention of the grant of the patent: **24.10.2012**
(21) Application number: 06733049.8
(22) Date of filing: 11.05.2006
(51) Int. Cl.: A61L 27/06, A61L 27/56, A61F 2/00, A61F 2/28, A61B 17/68, B29C 43/00

(54) **MOULDED ARTICLE AND MASS AND METHOD FOR PRODUCTION THEREOF**
GEFORMTE ARTIKEL UND MATERIAL UND VERFAHREN ZUR HERSTELLUNG DAVON
ARTICLE ET MASSE MOULES ET PROCEDES DE FABRICATION CORRESPONDANTS

(30) Priority: 11.05.2005 NL 1029017
(43) Date of publication of application: 27.02.2008
(73) Proprietor: Fondel Finance B.V., 3034 KA Rotterdam (NL)
(72) Inventor: MCLEOD, Allan Gordon, 3034 KA Rotterdam (NL)
(74) Representative: Hatzmann, Martin
(86) International application number: PCT/NL2006/000245
(87) International publication number: WO 2006/121330

(56) References cited:
- EP-A- 0 856 299
- WO-A-00/64504
- WO-A-2004/021905
- US-A- 5 716 359
- US-A1- 2003 193 104

## Description

The invention relates to moulded articles for use in a human or animal body. The invention especially but not exclusively relates to moulded articles for replacement or correction of body parts of human or animal bodies, specifically bone structures and parts thereof, or to means for attaching prosthesis.

In surgery, especially bone related surgery such as orthopaedic surgery, for example for replacements of joints such as placing of a hip prosthesis, operations on fractures or on the spinal cord, such as hernia operations, it is sometimes necessary to replace part of a body part, especially of a bone structure or part thereof, for example lost tissue due to cutting, bone resorbtion or tumours or to add material for support purposes. For this purpose it is known to use grafted material such as bone removed from an other part of the body or artificial body parts such as prosthesis.

Use of grafted material has the disadvantage that at least one second incision has to be made in a part of the body which is remote from the actual operation site to which said material is to be added. This may be traumatic to the patient, for example due to scar tissue forming after the operation and furthermore may increase the time to full recovery of the patient. This will also increase the risk of infections and lengthen the duration of the operation and thus of narcosis. Which should be kept to the minimum. Moreover, the graft material may be rejected or deteriorate over time.

Use of artificial body parts has the disadvantage that these may be rejected by the body, which means that relative large amounts of medicines may have to be used in order to minimise the risk of such rejection. Furthermore, such artificial body parts tend to loosen over time after the operation or the fit thereof deteriorates. Which means that a second or even further operation may become necessary. A further problem of these artificial body parts is that these are relative expensive and require removal of far more tissue from the body that required or desired for the actual operation.

US2003/0193104 biocompatible, bioabsorbable body implant, made of one or more layers of bioabsorbable polymeric foam provided on a reinforcement component. The article can be used for surgical repair of soft tissue injury, such as injury to the pelvic floor. A method for manufacturing this implant is described in which a solution of a desired polymeric material in a suitable solvent is added to a mould and the solution is lyophilised to obtain the implant in which a reinforcement material is embedded in a polymeric foam.

There is a need for a means for use in a human or animal body, for example for replacement or support of structures.

One object of the present invention is to provide a means for use in a human or animal body, which prevents or at least mitigates at least a number of the disadvantages of the known methods as discussed here above.

A further object of the present invention is to provide a moulded article, to be used in an animal or human body.

A still further object of the present invention is to provide a moulded article for us in a human or animal body, which enables bone or other body tissue ingrowth, which article is or can be formed conform a body space it is to be used in.

A further object of the present invention is to provide a mass for forming an article that can be used in an animal or human body, especially for replacement or support of a body part such as a bone structure or part thereof.

An even further object of the present invention is to provide for a method for forming an article for use in an animal or human body.

The invention also has as an object to provide for a method for replacing, filling and/or supporting part of a human or animal body such as a bone structure or part thereof.

At least a number of these and further objects of the invention can be met with the present invention.

According to the present invention there is provided a moulded article, comprising interconnected granules resulting in a rigid or semi rigid structure. The granules as such and the structure in general are biocompatible. At least part of the granules is porous.

A moulded article according to the present invention has the advantage that it can easily be made, in any desired shape, and can readily be placed and used inside a human or animal body. Due to the porosity of the structure of at least part of said granules bone and/or other bodily tissues can and will grow into said structure, in and/or onto said granules, such that a solid and permanent connection is obtained between said structure and the surrounding part of said body.

In the present application a moulded article has to be understood as any artefact having been made in any shape, by for example a moulding process in a mould, such as a compression mould or injection mould, or by "free moulding" in which a mass is shaped into a desired form without the use of a mould. This may also be referred to as "sculpturing" or similar terms. The term granules has to be understood as encompassing at least any relatively small element, having a regular or irregular shape. Porous has to be understood as having at least openings going into and/or through said granules and/or said structure.

In a preferred embodiment the granules are at least interconnected by mechanical interaction, especially but not exclusively due to pressure.

A moulded article according to the present invention can be moulded in a mould, defining a general shape of said structure. Within said mould a physical and/or chemical reaction can occur for forming the interconnection between the granules, which can be controlled well. A mould to be used for forming a moulded article according to the present invention may be of solid and fixed shape design. However, such mould can also be partly or fully of a flexible design, for example made of an elastomeric or rubbery material or provided with movable parts, inserts or the like in order to amend the shape of the mould, especially a mould cavity within said mould, to fit the desired use of the moulded article.

Preferably the moulded article, especially the structure thereof, is at least partly porous, such that bone tissue and/or bodily fluids of a human or animal can pass through and/or grow into at least part of said article. This will result in a direct connection of the article to a bodily structure such as bone.

The granules are preferably made of or at least containing titanium or a titanium alloy. Most preferably the granules comprise reaction products of titanium chloride resulting in a desired sponge-like structure. This will lead to a structure having improved ingrowth of body tissue, especially bone, and better controlled porosity when compared to for example granules made by blowing gas through molten titanium. However, other chemical and/or physical methods may be used for obtaining porosity in granules. The granules preferably have a sponge like structure, having relatively large pores between thin wire like elements.

The granules may be vibrated prior to or during interconnection. This results in a closer and more tight packing of the granules and better connecting. The density of the resulting moulded article can be varied by inter alia said vibrating.

In an alternative embodiment the moulded article is formed out of a deformable mass comprising said granules. Said mass preferably is or has been kneadable such that it can be sculptured into the desired shape. To this end the mass from which the article is to be formed may comprise a compound holding together said granules, for example a binding agent, which may be removed, for example by rinsing after forming said article, or can be used as a hardening agent for interconnecting said granules and forming said rigid structure. Said compound is chosen such that the porosity of the granules and/or structure is maintained or can be easily restored, for example by said rinsing with a fluid.

The outer surface of the structure is preferably at least partly and more preferably largely defined by the outer surfaces of granules, such that it has a roughness larger than the roughness of the individual granules. This may further improve the attachment of bodily matter such as bone and other tissue to said structure. The granules may be coated such as an adhesive coating.

The invention further relates to a mass for forming an article according to the present invention, comprising granules having at least partly a porous structure. The mass preferably is a heat settable mass.

The granules may be in a size range of for example between 0.2 and 10 mm and may be chosen based on inter alia the desired use. For example, in femur related applications preferably sizes are used between 0.5 and 2 mm, in acetabulum and cavity filling related applications preferably sizes are used between 0.5 and 2 mm.

The present invention further relates to a method for forming a moulded article, characterised by the features of claim 19.

A method according to the present invention has the advantage that a moulded article can be obtained for use in a human or animal body, for replacement of or support of a bodily structure or part thereof.

In an alternative, the present invention relates to an article for replacement of a body part, characterised by the features of claim 24.

Replacement of a body part has to be understood as encompassing but not limited to filling openings or holes in a bodily structure such as part of a skeleton, replacement of a complete bone or bone structure such as a vertebrae or an inter vertebrae disc or support of a body part or a prosthesis, for example in stead of or additional to cement in fixture of a prosthesis.

In a further embodiment the present invention relates to the use of granules made of bio compatible material, as disclosed in claim 26.

The dependent claims disclose further advantageous embodiments of the present invention, which should not be considered limiting the scope of the present invention. Especially embodiments or aspects in the description should be considered as disclosed and claimed also.

In order to understand the present invention more fully embodiments thereof will be shown and discussed, with reference to the drawings. These show:
fig. 1 in cross section schematically a mould containing a mass comprising granules according to the present invention;
fig. 2 schematically a moulded article according to the present invention for use as a disc to be inserted between vertebrae;
fig. 3 schematically an article according to fig. 2 with sections having different densities;
fig. 4 schematically a prosthesis in a receiving opening in a human femur, articles according to the present invention having been inserted between the femur and the inside wall of said receiving opening;
fig. 5 schematically in perspective view an article as used in an assembly according to fig. 4; and
fig. 6 in cross section schematically a granule of or in an article or mass according to the present invention.

These embodiments are given as examples only and should not be considered as limiting the invention in any way. In the description and drawings the same or corresponding items or features are designated by the same or similar reference signs. Combinations of features of the various embodiments shown are considered disclosed herein also.

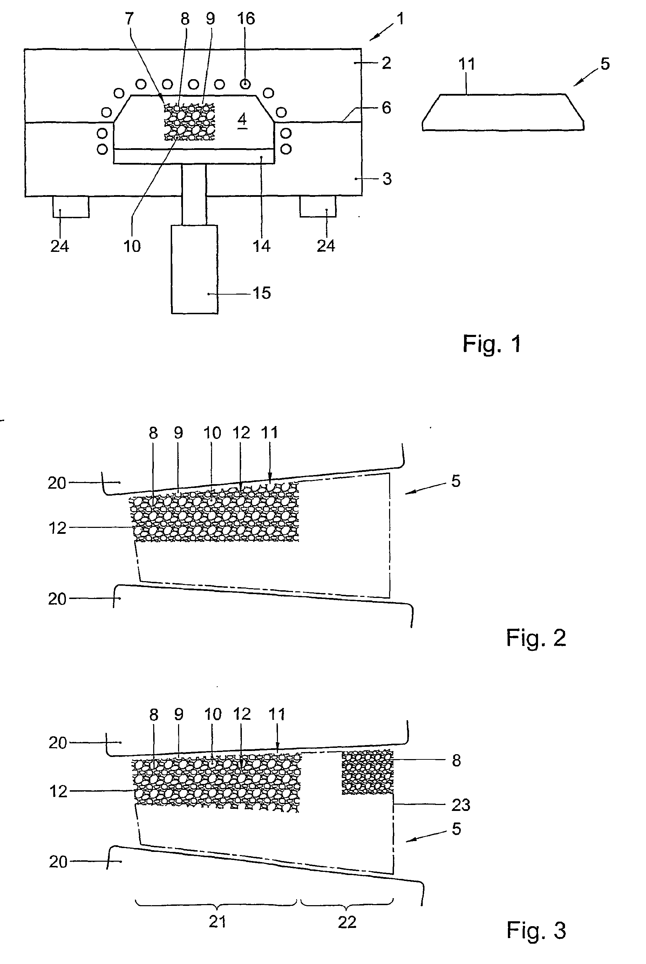

In fig. 1 a mould 1 is shown having an upper mould halve 2 and a lower mould halve 3. The mould halves 2, 3 enclose and define between them a mould cavity 4 having the shape of an article 5 to be formed therein. Such article 5 is shown schematically next to said mould 1. In the present form said article 5 is for example in the shape of (part of) a bone or bone structure to be replaced or augmented, supported or otherwise provided with or replaced by said article 5.

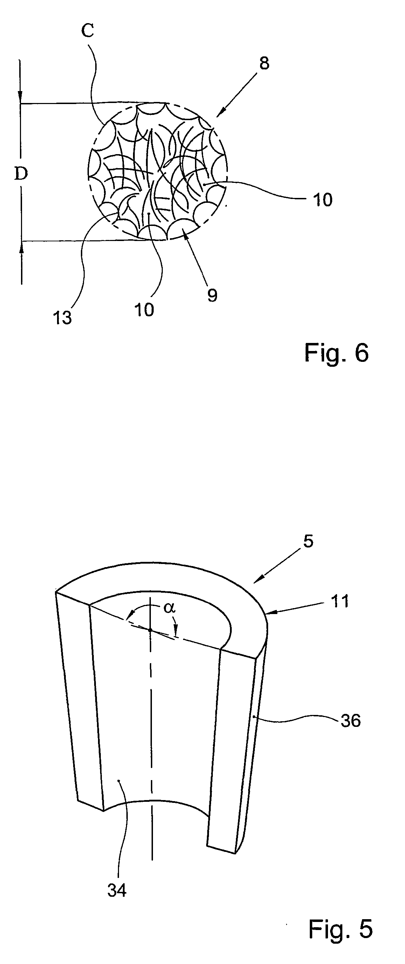

The mould halves 2, 3 can be opened by separating them along a dividing line. 6, so as to fill the cavity 4 or remove an article there from. In fig. 1 a mass 7 has been introduced into said cavity 4, which mass 7 comprises granules 8 having an irregular shape and outer surface 9. Only part of the mass 7 is shown. The granules 8, as shown more specifically in fig. 6, are at least partly and preferably entirely porous, having openings 10 (shown as dots and/or surface irregularities and/or open spaces) in said surface 9. Said openings 10 may be formed as an irregularly shaped and dimensioned network of openings 10 extending through said granules 8, such that a sponge like configuration is obtained between the granule material 13. The granules 8 preferably have a porous configuration such that bodily tissue of a human or animal body can grow into and/or through said granules. For example bone can grow into said granules and blood can flow into and through said openings in said granules and/or adhere to or grow into said surface 9.

In the mould 1 as shown in fig. 1 the lower mould part 3 comprises a movable wall part 14, form example a piston, which can be forced into said cavity 4, into or against the mass 7, by a forcing member such as a hydraulic or pneumatic cylinder 15. By means of said movable wall part 14 the volume of the cavity 4 can be reduced, to the actual desired shape and volume of the cavity conform the article 5 to be made, thereby compressing the mass 7, which prior to said compression filled the cavity 4. By said compression the granules 8 may be forced against and even partly into each other, providing an interconnection between said granules 8 in said mass 7. Furthermore heating means 16 may be provided in said mould 1, especially around said cavity 4, for heating the mass 7 inside said cavity, before, during and/or after said compression. By said compression and/or said heating the granules in said mass 7 may be further interconnected. If applied heating is preferably restricted to temperatures below approximately 40 °C

In an other embodiment the mass 7 comprises a settable compound such as collagen powder, hydroxyappatite powder, preferably settable at temperatures below 40 °C which will, due to at least compression, interconnect said granules 8 in said mass 7. In a preferred embodiment the granules are compressed such that they are mechanically interconnected, preferably without any further compound as described above.

It should be noted that a mould 1 could also be used according to the present invention without the movable wall part 14. The mass 7 can be such that a chemical or physical interconnection can be obtained between the granules, using a compound as described above in said mass, for example a settable adhesive, two or more compound adhesives or the like. Also a mould 1 according to fig. 1 can be used without the heating means, when the pressure build up due to the compacting or compression provides for sufficient interconnection between the granules.

The granules 8 in said article 5 as for example shown in fig. 1-5 are interconnected. They are connected to each other such that the article is or has a rigid or semi rigid structure. This means that the article is self containing and can thus be manipulated in one piece, without the necessity of an outer package or cover to encage said granules. The outer surface 11 of the structure or article 5 is therefore in the embodiments shown basically defined by the outer surfaces 9 of the granules 8. The interconnection can be provided by parts of the surfaces 9 of granules 8 interacting, for example by entanglement of protrusions of said granules. The granules 8 could also be interconnected by a compound as described above, under relatively low heat and/or pressure. Part or all of the surface 9 of part or all of the granules may be provided with an adhesive, for example a pressure sensitive or temperature sensitive glue or melt, for obtaining or improving the desired interconnection. In a further preferred embodiment the speed of compression is chosen relatively high, in order to avoid negative visco elastic effects.

The surface roughness of the surface 11 of the article 5, formed by the granules, can be higher than the surface roughness of the individual granules 8. Between the granules 8 within said article 5 pores 12 can be formed providing further passage ways for bodily tissue such as bone or blood to enter into said article and/or pass through and/or grow into said article. The article in the embodiment shown is therefore porous both resulting from porosity of the granules and of the structure

The granules are made of a biocompatible material, such as metal, and are preferably made porous by chemical reaction or by blowing gas through a liquefied material such as metal or ceramics. In a preferred embodiment the granules are reaction products of a reaction of titanium chloride, resulting in said desired sponge like structure. By control of said reaction, for example through dosage of said chloride relative to the amount of titanium, the porosity can be controlled accurately. The granules 8 can have any desired shape, size and porosity, as well as density. In this description the size of a granule is defined by the diameter C of the smallest sphere into which said granule can fit, as is schematically shown in fig. 6.

In fig. 2 an article 5 according to the present invention is shown in side view, in the shape of a disc for positioning between vertebrae 20 of a human or animal. The disc in the present embodiment has a wedge shaped side view and for example a rectangular top and bottom view, perpendicular to the surface of the drawing. The disc is in the present embodiment entirely made of granules 8, especially titanium granules. The outer surface 11 is entirely defined by the granules 8. The disc has a porous structure, comprising said pores 12 and openings 10 through said granules. During use bone can grow into said pores 12 and onto and/or into said granules 8, leading to a solid connection between the article 5 and the vertebrae 20. During at least said ingrowth of said bone blood and other bodily fluids can pass through said article 5.

In fig. 3 an article 5 similar to the article 5 in fig. 2 is shown. However, in this embodiment a first section 21 of the article 5 has a density of granules 8, which is lower than the density of granules in a second section 22. In the embodiment shown the second section 22 is positioned at a dorsal end 23 of the article 5. This can for example be obtained by higher compression of the second section and/or use of smaller granules 8. The higher density may lead to a better resistance to further compression and a higher load bearing capacity. This can also be obtained by a non-homogenous distribution of a connecting compound as discussed before throughout the article.

When a compound for providing said interconnection between the granules is used, this is preferably chosen such that any excess compound can easily be removed, for example by rinsing said article with a suitable solvent, such that the desired porosity of the article is obtained. Most preferably such compound is chosen such that it does not enter into the openings of the granules.

As is shown in fig. 1, the mould can be provided with vibrating means 24, shown as vibration mats. These can vibrate the mould 1 when the mass 7 is in the cavity, leading to a further compacting of the mass. This will result in a higher density of the article and to a further improved interconnection of the granules.

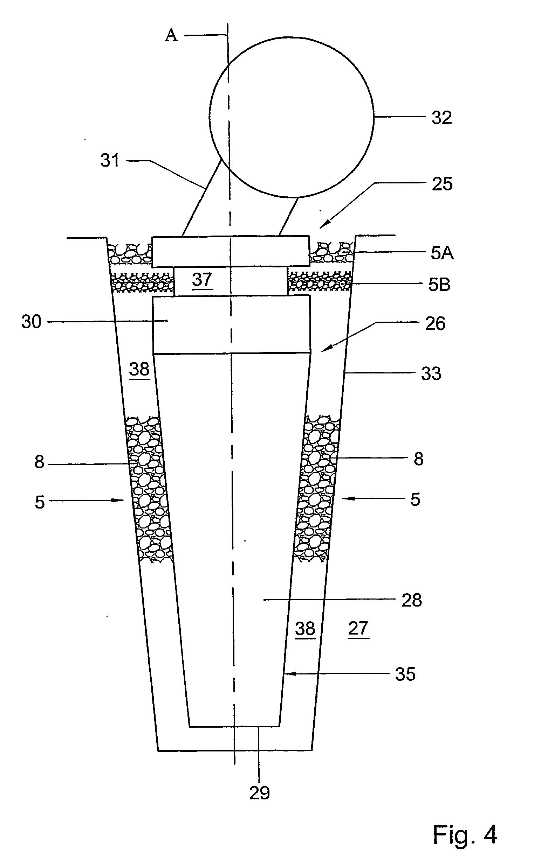

Fig. 4 shows in sectional side view a femoral component 25 of a hip prosthesis in an opening 26 in a femur 27. This hip prosthesis is only shown by way of example and should not be understood as limiting the invention to such prosthesis. The femoral component 25 comprises a shaft 28, which tapers toward a free lower end 29. At the opposite end a shoulder 30 is provided, from which a neck 31 extends. A spherical head 32 is carried by the neck 31, for interacting with a acetabulum component (not shown) Such basic designs of femoral component are generally known in the art and are normally fitted into said opening 26 in said femur 27 by using cement inserted between said shaft 28 and the interior wall 33 of the opening 26. Cement can be disadvantageous since it may not bear the load for a sufficient time and will take time to set.

According to the present invention as shown in fig. 5, moulded articles 5 are positioned between the shaft 26 and said interior wall 33, said moulded articles 5 being articles 5 according to the present invention, comprising interconnected granules. The articles 5 may have been "baked", that is heated at a relatively low temperature, for example below approximately 40 °C in a mould 1 under pressure. In fig. 6 the outer contours of such article 5 are shown as positioned between said shaft and said interior wall 33. The articles 5 are shaped as cylinder segments having an inside wall 34, which is complementary to a part of the outer surface 35 of the shaft 28 to which it is to be positioned. The outer surface 36 is substantially complementary to the part of the interior wall 33 of the opening 26 against which it is to rest. The article 5 has a height that may be smaller than the length of the shaft but it may also be similar in height. The segments may enclose an angle α such that a discrete number of articles 5 can be positioned around said shaft, forming a substantially full circle. In the embodiment shown the angle α is approximately 180° so that two articles 5 are positioned around said shaft. The angle could however also be for example 45°, 60° or 90° whereas also articles 5 can be used having different angles α, whereas also articles can be combined having different densities, porosities, types of granules, lengths or configurations, for example in order to provide different supports to different sides of said shaft. By the tapered form of the article 5 a good positioning of the shaft 28 can easily be obtained. Due to the rigid structure of the article instant support can be obtained.

In the embodiment of fig. 4 the rest of the annular space 38, not filled by the moulded articles 5 discussed above can be filled with for example loose granules similar to the granules 8 as used in the articles 5 but also cement could be used as commonly used for cementing prosthesis. Also bodily tissue such as bone can be inserted in said space 36. In a preferred embodiment further articles according to the present invention, designated 5A, are inserted in at least part of said space 38, for example between the shoulder 30 and the interior wall 33. In the shoulder 30 an annular groove 37 may be provided from which further articles according to the invention, designated 5B can be provided, locking the shaft 28 even better in position.

It shall be clear to any person skilled in the art that articles can be varied in for example the shape, number, type, sizes, granules used, compounds added and the like depending on for example the position and function it will have to get inside a human or animal body.

According to the present invention a mass 7 can be used which is kneadable. This means that the rheology of the mass 7 is such that it can be shaped, especially sculptured manually, without using a closed mould as shown in fig. 1. The mass is at least initially highly viscous, such that it can be moulded by hand into any desired shape, after which it can set, for example thermically or chemically, into a more rigid structure. In the kneadable form it is referred to as semi-rigid. Such mass has the advantage that it can be shaped for example during an operation, without difficult tools, depending on the desired use. For example cuts or openings in or between bones in a skeleton can be filled, such that during healing bone will grow into said article.

The granules preferably have an outer surface which is coarse in machining, resulting in a relatively high surface roughness of for example over 6 Ra, more specifically over 6.3 Ra. The products produced according to the present invention may have an even higher roughness. Granules of other materials having porosity could be used, for example hydroxiapatite. The porosity is preferably such that body fluids can enter the granules. Most preferably the pores are at least partly interconnected, such that said body fluids can pass through, promoting bone growth into said granules.

The present invention is by no means limited to the embodiments shown and discussed by way of example only. Many variations are possible, including all combinations and variations of different aspects of the embodiments shown.

Articles according to the invention can have other shapes and dimensions, can be made of different granules of different materials and can comprise solid parts, for example a core of soiled or hollow metal, plastic or natural material, covered with a layer of granules as disclosed in the present description. Chemical substances such as medicines can be added to said granules or in between said granules and/or in such core, for example to increase acceptance of the article, promote bone growth into and adherence of bone to said article and the like. Part of the granules can be made bio degradable, such that during ingrowth of bone into said article, part of the article dissolves into the body of relevant human or animal. This may improve recovery and acceptance even further. Articles according to the present invention can be used inside the animal or human body but could also be used outside such body, for example for training or exhibition purposes.

## Claims

1. Moulded article (5), comprising granules (8) made of a bio compatible material, which granules (8) have a substantially porous structure and are interconnected, forming a rigid or semi-rigid structure.

2. Moulded article (5) according to claim 1, wherein said granules (8) are interconnected by at least a chemical compound.

3. Moulded article (5) according to claim 1 or 2, wherein said granules (8) are interconnected at least by mechanical interaction due to pressure.

4. Moulded article (5) according to any one of the preceding claims, wherein the article (5) has been shaped in a mould (1).

5. Moulded article (5) according to any one of the preceding claims, wherein said article (5) has a porous structure, such that bone tissue and/or bodily fluids of a mammal can pass through at least part of said article.

6. Moulded article (5) according to any one of the preceding claims, wherein said granules (8) comprise titanium or titanium alloy.

7. Moulded article (5) according to any one of the preceding claims, wherein said granules (8) have a sponge like structure.

8. Moulded article (5) according to any one of the preceding claims, wherein the granules (8) have been vibrated prior to or during interconnecting these to form said structure.

9. Moulded article (5) according to any one of the preceding claims, wherein said structure is semi-rigid, such that it is manually kneadable.

10. Moulded article (5) according to any one of the preceding claims, wherein said structure has an outer surface (11), at least partly formed by said granules (8), such that the roughness of said surface (11) is higher then the roughness of the individual granules (8).

11. Moulded article (5) according to any one of the preceding claims, wherein the surface (9) of at least a number of said granules (8) is provided with a coating, especially an adhesive compound.

12. Mass (7) comprising granules (8) for forming a moulded article (5) according to any one of the preceding claims.

13. Mass (7) according to claim 12, wherein said mass (7) contains granules (8) made of titanium or titanium alloy, wherein the granules (8) preferably are reaction products of a reaction of titanium chloride, resulting in said desired sponge like structure.

14. Mass (7) according to claim 12 or 13, wherein said mass (7) comprises granules (8) having an average diameter of between 0.2 and 10 mm.

15. Mass (7) according to any one of claims 12 -14, wherein the granules (8) in said mass (7) have an average diameter between 0.5 and 7 mm.

16. Mass (7) according to any one of claims 12 - 15, wherein at least part of said granules (8) is provided with a coating, especially an adhesive coating.

17. Mass (7) according to any one of claims 12 - 16, wherein said mass (7) comprises at least one compound for providing for or improving interconnection of said granules (8), for example under pressure or heating or by chemical or physical reaction.

18. Mass (7) according to any one of claims 12 - 17, wherein said mass (7) is kneadable and preferably settable to a rigid structure, for example under pressure or heating or by chemical or physical reaction.

19. Method for forming a moulded article, comprising the steps of;
- providing a mass (7) comprising at least granules (8) made of a bio compatible material and having a porous structure;
- pressing said mass (7), especially said granules together, for forming a rigid or semi rigid structure.

20. Method according to claim 19, wherein said pressing is performed in a mould (1).

21. Method according to claim 19 or 20, wherein during at least part of said pressing said mass (7) is heated, preferably to a temperature below approximately 40 °C.

22. Method according to any one of claims 19 - 21, wherein said mass (7) is vibrated prior to and/or during at least part of said pressing.

23. Method according to any one of claims 19 - 22, wherein the article (5) is shaped conform a body part of a human or animal, especially a bone structure or part thereof to be replaced.

24. Article (5) for replacement of a body part of a human or animal, especially a bone structure or part thereof, comprising interconnected granules (8), said granules (8) having a substantially porous structure and made of bio compatible material.

25. Article (5) according to claim 24, wherein the granules form a rigid or semi-rigid, porous structure.

26. Use of granules (8) made of bio compatible material and a substantially porous configuration, for preparing a mass (7) for replacement of a body part of a human or animal, especially a bone structure.

27. Use according to claim 26, wherein said particles in said mass (7) are interconnected, such that they can be manipulated as one artefact, without said granules (8) falling out.

## Patentansprüche

1. Formteil (5), umfassend Körnchen (8) aus einem biokompatiblen Material, wobei die Körnchen (8) eine im Wesentlichen poröse Struktur aufweisen und miteinander verbunden sind, wobei sie eine starre oder halbstarre Struktur bilden.

2. Formteil (5) nach Anspruch 1, wobei die Körnchen (8) durch mindestens eine chemische Verbindung miteinander verbunden sind.

3. Formteil (5) nach Anspruch 1 oder 2, wobei die Körnchen (8) mindestens durch mechanische Wechselwirkung infolge von Druck miteinander verbunden sind.

4. Formteil (5) nach einem der vorhergehenden Ansprüche, wobei das Teil (5) in einer Form (1) gebildet wurde.

5. Formteil (5) nach einem der vorhergehenden Ansprüche, wobei das Teil (5) eine poröse Struktur aufweist, so dass Knochengewebe und/oder Körperflüssigkeiten eines Säugetiers durch mindestens einen Teil des Teils dringen können.

6. Formteil (5) nach einem der vorhergehenden Ansprüche, wobei die Körnchen (8) Titan oder eine Titanlegierung umfassen.

7. Formteil (5) nach einem der vorhergehenden Ansprüche, wobei die Körnchen (8) eine schwammartige Struktur aufweisen.

8. Formteil (5) nach einem der vorhergehenden Ansprüche, wobei die Körnchen (8) vor oder während ihrer Verbindung zum Bilden der Struktur vibriert wurden.

9. Formteil (5) nach einem der vorhergehenden Ansprüche, wobei die Struktur halbstarr ist, so dass sie von Hand knetbar ist.

10. Formteil (5) nach einem der vorhergehenden Ansprüche, wobei die Struktur eine Außenoberfläche (11) aufweist, die mindestens teilweise durch die Körnchen (8) gebildet wird, sodass die Rauheit der Oberfläche (11) höher ist als die Rauheit der einzelnen Körnchen (8).

11. Formteil (5) nach einem der vorhergehenden Ansprüche, wobei die Oberfläche (9) von mindestens einer Anzahl der Körnchen (8) mit einer Beschichtung versehen ist, insbesondere einem Klebstoff.

12. Masse (7), umfassend Körnchen (8) zum Bilden eines Formteils (5) nach einem der vorhergehenden Ansprüche.

13. Masse (7) nach Anspruch 12, wobei die Masse (7) Körnchen (8) enthält, hergestellt aus Titan oder einer Titanlegierung, wobei die Körnchen (8) vorzugsweise Reaktionsprodukte einer Reaktion von Titanchlorid sind, bei der die gewünschte schwammähnliche Struktur entsteht.

14. Masse (7) nach Anspruch 12 oder 13, wobei die Masse (7) Körnchen (8) mit einem durchschnittlichen Durchmesser zwischen 0,2 und 10 mm umfasst.

15. Masse (7) nach Anspruch 12 - 14, wobei die Körnchen (8) in der Masse (7) einen durchschnittlichen Durchmesser zwischen 0,5 und 7 mm umfasst.

16. Masse (7) nach einem der Ansprüche 12 - 15, wobei mindestens ein Teil der Körnchen (8) mit einer Beschichtung versehen ist, insbesondere einer Klebebeschichtung.

17. Masse (7) nach einem der Ansprüche 12 - 16, wobei die Masse (7) mindestens eine Verbindung umfasst, um die Verbindung der Körnchen (8) zum Beispiel unter Druck, durch Erwärmen oder durch eine chemische oder physikalische Reaktion zu bilden oder zu verbessern.

18. Masse (7) nach einem der Ansprüche 12 - 17, wobei die Masse (7) knetbar ist und vorzugsweise zu einer starren Struktur setzbar ist, beispielsweise unter Druck, durch Erwärmen oder durch eine chemische oder physikalische Reaktion.

19. Verfahren zum Herstellen eines Formteils, umfassend die Schritte:
- Bilden einer Masse (7), umfassend mindestens Körnchen (8), die aus einem biokompatiblen Material hergestellt sind und eine poröse Struktur aufweisen;
- Zusammenpressen der Masse (7), insbesondere der Körnchen, um eine starre oder halbstarre Struktur zu bilden.

20. Verfahren nach Anspruch 19, wobei der Pressvorgang in einer Form (1) ausgeführt wird.

21. Verfahren nach Anspruch 19 oder 20, wobei während mindestens eines Teils des Pressvorgangs die Masse (7) erwärmt wird, vorzugsweise auf eine Temperatur unter ungefähr 40°C.

22. Verfahren nach einem der Ansprüche 19 - 21, wobei die Masse (7) vor und/oder während mindestens einem Teil des Pressvorgangs vibriert wird.

23. Verfahren nach einem der Ansprüche 19 - 22, wobei das Teil (5) nach einem Körperteil eines Menschen oder eines Tiers geformt ist, insbesondere einer Knochenstruktur oder eines Teils davon, die bzw. der ersetzt werden soll.

24. Teil (5) zum Ersetzen eines Körperteils von einem Menschen oder einem Tier, insbesondere einer Knochenstruktur oder eines Teils davon, umfassend miteinander verbundene Körnchen (8), wobei die Körnchen (8) eine im Wesentlichen poröse Struktur aufweisen und aus einem biokompatiblen Material hergestellt sind.

25. Teil nach Anspruch 24, wobei die Körnchen eine starre oder halbstarre poröse Struktur bilden.

26. Verwendung von Körnchen (8), hergestellt aus einem biokompatiblen Material und von einer im Wesentlichen porösen Anordnung zur Herstellung einer Masse (7) zum Ersetzen eines Körperteils von einem Menschen oder einem Tier, insbesondere einer Knochenstruktur.

27. Verwendung nach Anspruch 26, wobei die Teilchen in der Masse (7) miteinander verbunden sind, so dass sie als ein Produkt bearbeitet werden können, ohne dass die Körnchen (8) herausfallen.

## Revendications

1. Article moulé (5), comprenant des granules (8) faits d'un matériau biocompatible, les granules (8) ayant une structure essentiellement poreuse et étant interconnectés, en formant une structure rigide ou semi-rigide.

2. Article moulé (5) selon la revendication 1, dans lequel lesdits granules (8) sont interconnectés par au moins un composé chimique.

3. Article moulé (5) selon la revendication 1 ou 2, dans lequel lesdits granules (8) sont interconnectés au moins par interaction mécanique due à une pression.

4. Article moulé (5) selon l'une quelconque des précédentes revendications, dans lequel l'article (5) a été formé dans un moule (1).

5. Article moulé (5) selon l'une quelconque des précédentes revendications, dans lequel ledit article (5) a une structure poreuse, de sorte qu'un tissu osseux et/ou des fluides corporels d'un mammifère peuvent passer à travers au moins une partie dudit article.

6. Article moulé (5) selon l'une quelconque des précédentes revendications, dans lequel lesdits granules (8) comprennent du titane ou un alliage de titane.

7. Article moulé (5) selon l'une quelconque des précédentes revendications, dans lequel lesdits granules (8) ont une structure de type spongieux.

8. Article moulé (5) selon l'une quelconque des précédentes revendications, dans lequel lesdits granules (8) ont été soumis à des vibrations avant ou pendant leur interconnexion afin de former ladite structure.

9. Article moulé (5) selon l'une quelconque des précédentes revendications, dans lequel ladite structure est semi-rigide, de sorte qu'elle peut être malaxée manuellement.

10. Article moulé (5) selon l'une quelconque des précédentes revendications, dans lequel ladite structure comporte une surface extérieure (11), au moins en partie formée par lesdits granules (8), de sorte que la rugosité de ladite surface (11) est supérieure à la rugosité des granules individuels (8).

11. Article moulé (5) selon l'une quelconque des précédentes revendications, dans lequel la surface (9) d'au moins un certain nombre desdits granules (8) est munie d'un revêtement, notamment un composé adhésif.

12. Masse (7) comprenant des granules (8) pour former un article moulé (5) selon l'une quelconque des précédentes revendications.

13. Masse (7) selon la revendication 12, dans laquelle ladite masse (7) contient des granules (8) faits de titane ou d'un alliage de titane, dans laquelle les granules (8) sont de préférence les produits de réaction d'une réaction de chlorure de titane, résultant en ladite structure de type spongieux voulue.

14. Masse (7) selon la revendication 12 ou 13, dans laquelle ladite masse (7) comprend des granules (8) ayant un diamètre moyen de 0,2 à 10 mm.

15. Masse (7) selon l'une quelconque des revendications 12 à 14, dans laquelle les granules (8) de ladite masse (7) ont un diamètre moyen de 0,5 à 7 mm.

16. Masse (7) selon l'une quelconque des revendications 12 à 15, dans laquelle au moins une partie desdits granules (8) est munie d'un revêtement, notamment un composé adhésif.

17. Masse (7) selon l'une quelconque des revendications 12 à 16, dans laquelle ladite masse (7) comprend au moins un composé pour permettre ou pour améliorer l'interconnexion desdits granules (8), par exemple, sous pression ou par chauffage ou par réaction chimique ou physique.

18. Masse (7) selon l'une quelconque des revendications 12 à 17, dans laquelle ladite masse (7) peut être malaxée et peut être de préférence durcie en une structure rigide, par exemple, sous pression ou par chauffage ou par réaction chimique ou physique.

19. Procédé de formation d'un article moulé, comprenant les étapes consistant à :
prendre une masse (7) comprenant au moins des granules (8) faits d'un matériau biocompatible et ayant une structure poreuse ;
comprimer ladite masse (7), notamment lesdits granules ensemble, afin de former une structure rigide ou semi-rigide.

20. Procédé selon la revendication 19, dans lequel ladite compression est effectuée dans un moule (1).

21. Procédé selon la revendication 19 ou 20, dans lequel, durant au moins une partie de ladite compression, ladite masse (7) est chauffée, de préférence à une température inférieure à environ 40°C.

22. Procédé selon l'une quelconque des revendications 19 à 21, dans lequel ladite masse (7) est soumise à des vibrations avant et/ou pendant au moins une partie de ladite compression.

23. Procédé selon l'une quelconque des revendications 19 à 22, dans lequel l'article (5) est formé de manière à épouser la forme d'une partie corporelle d'un être humain ou d'un animal, notamment une structure osseuse ou une partie de celle-ci devant être remplacée.

24. Article (5) destiné à remplacer une partie corporelle d'un être humain ou d'un animal, notamment une structure osseuse ou une partie de celle-ci, comprenant des granules interconnectés (8), lesdits granules (8) ayant une structure essentiellement poreuse et étant faits d'un matériau biocompatible.

25. Article (5) selon la revendication 24, dans lequel les granules forment une structure poreuse rigide ou semi-rigide

26. Utilisation de granules (8) faits d'un matériau biocompatible et ayant une configuration essentiellement poreuse, pour préparer une masse (7) destinée à remplacer une partie corporelle d'un être humain ou d'un animal, notamment une structure osseuse.

27. Utilisation selon la revendication 26, dans laquelle lesdites particules de ladite masse (7) sont interconnectées, de sorte qu'elles peuvent être manipulées comme un artefact, sans que lesdits granules (8) ne tombent.
